# EUROPEAN PATENT SPECIFICATION

(11) **EP 4 111 969 B1**
(45) Date of publication and mention of the grant of the patent: **08.04.2026**
(21) Application number: 21761536.8
(22) Date of filing: 26.02.2021
(51) Int. Cl.: A61B 5/00, A61B 5/145, A61B 5/1486

(54) **SENSOR AND METHOD FOR MANUFACTURING SAME**
SENSOR UND VERFAHREN ZU DESSEN HERSTELLUNG
CAPTEUR ET SON PROCÉDÉ DE FABRICATION

(30) Priority: 28.02.2020 US 202062982939 P
(43) Date of publication of application: 04.01.2023
(73) Proprietor: PHC Holdings Corporation, Tokyo 100-8403 (JP)
(72) Inventor: EZAKI, Hirofumi, Ehime 791-0395 (JP); SAKAMOTO, Isao, Ehime 791-0395 (JP); FUJIWARA, Masaki, Ehime 791-0395 (JP)
(74) Representative: Eisenführ Speiser
(86) International application number: PCT/JP2021/007510
(87) International publication number: WO 2021/172561

(56) References cited:
- WO-A1-2017/187943
- WO-A1-2019/006413
- CN-A- 105 411 607
- JP-A- 2019 170 701
- JP-A- 2019 514 615
- US-A1- 2010 230 285
- US-A1- 2019 310 218

## Description

### Technical Field

The present disclosure relates to a sensor and a method for manufacturing the same.

### Background Art

US 2010/230285 A1 refers to an analyte sensor, comprising a substrate; a conductive layer disposed over at least a portion of the substrate; a dielectric layer disposed over the conductive layer and having a void therein; and a sensing layer disposed within the void, wherein the area of the sensing layer in contact with the conductive layer has a sensor-to-sensor coefficient of variation of less than approximately 5% within a sensor lot.
CN 105 411 607 A provides a microsensor, and in particular relates to a subcutaneous tissue involvement type glucose microsensor and a preparation method thereof. The subcutaneous tissue involvement type glucose microsensor comprises a base, wherein the base is internally provided with a lead part extending outwards, the tail end of the lead part is connected with a sensor part, and the sensor part and the lead part are arranged on an insulation substrate layer.
Representative examples of electrochemical biosensors using enzyme include an electrochemical glucose sensor used for self-monitoring of blood glucose. Also, an embedded electrochemical glucose sensor for continuously or semi-continuously measures the concentration of glucose in a living body has been developed (see PTL 1, for example).

### Citation List

### Patent Literature

PTL 1
Japanese Unexamined Patent Application Publication (Translation of PCT Application) No. 2012-519038

### Summary of Invention

### Technical Problem

An electrochemical sensor for measuring an analyte such as glucose is required to perform measurement with higher accuracy.

A non-limiting example of the present disclosure provides a sensor capable of measuring an analyte with higher accuracy and a method for manufacturing the same.

### Solution to Problem

A sensor according to an example of the present disclosure is a sensor as defined in claim 1.

A method for manufacturing a sensor according to an example of the present disclosure is a method as defined in claim 7.

### Advantageous Effects of Invention

According to an example of the present disclosure, it is possible to measure an analyte with higher accuracy.

Further advantages and effects of the example of the present disclosure will become obvious from the specification and the accompanying drawings. Although each of such advantages and/or effects will be provided by some embodiments and features described in the specification and the accompanying drawings, it is not necessary that all the advantages and/or the effects be provided to obtain one or more same features.

### Brief Description of Drawings

FIG 1 illustrates an application example of a sensor according to Embodiment 1;
FIG 2 illustrates a sectional view of the sensor;
FIG 3 illustrates plan views of a probe;
FIG 4A illustrates a sectional view along arrow AA in FIG 3;
FIG 4B illustrates a sectional view along arrow BB in FIG 3;
FIG 4C illustrates a sectional view along arrow CC in FIG 3;
FIG 5 describes a positional relationship between a reagent layer and a film;
FIG 6 illustrates a sectional view along arrow DD in FIG 5;
FIG 7 illustrates a perspective view of a reagent layer part of the probe;
FIG 8 illustrates a plan view of a distal end part of the probe;
FIG 9 describes the positional relationship between the reagent layer and the film;
FIG 10 illustrates a sectional view along arrow EE in FIG 9;
FIG 11 illustrates examples of an opening shape of the film;
FIG 12 describes an example of a sensor size;
FIG 13 illustrates a perspective view of a probe of a sensor according to Embodiment 2;
FIG 14 illustrates a partial side view of the probe in FIG 13 when seen from a third surface side;
FIG 15 describes shape examples of reference layer 24;
FIG 16 illustrates an application example of an Ag/AgCl paste;
FIG 17 illustrates a side view after the Ag/AgCl paste is applied;
FIG 18A describes an example of a method for manufacturing the probe;
FIG 18B describes the example of the method for manufacturing the probe;
FIG 18C describes the example of the method for manufacturing the probe;
FIG 19 describes application of the Ag/AgCl paste;
FIG 20 describes the shape after the Ag/AgCl paste is applied;
FIG 21 describes operations of an application apparatus;
FIG 22 describes operations of the application apparatus;
FIG 23 illustrates shape examples of a reference layer obtained by a single-application technique;
FIG 24 illustrates shape examples of the reference layer obtained by a repeated-application technique; and
FIG 25 illustrates shape examples of the reference layer obtained by a squeegee technique.

### Description of Embodiments

Hereinafter, embodiments of the present disclosure will be described in detail with reference to the accompanying drawings as needed. However, there may also be a case in which unnecessarily detailed description is omitted. For example, there may be a case in which detailed description of matters that have already been known well and repeated description of substantially the same configurations are omitted. This is for avoiding the following description from becoming unnecessarily redundant and for facilitating understanding of those skilled in the art.

Note that the accompanying drawings and the following description are provided to allow those skilled in the art to sufficiently understand the present disclosure and are not intended to thereby limit the subject matter described in the claims.

### (Embodiment 1)

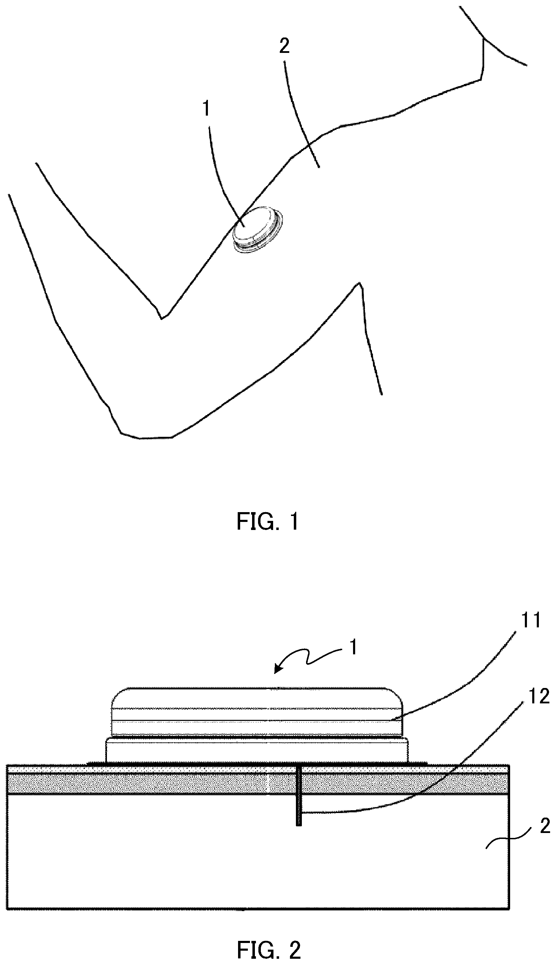

FIG 1 illustrates an application example of sensor 1 according to Embodiment 1. In FIG 1, living body 2 is illustrated as well as sensor 1. Living body 2 is, for example, a human body.

Sensor 1 illustrated in FIG 1 is, for example, a biosensor. More specifically, sensor 1 is a CGM (Continuous Glucose Monitor) sensor. Sensor 1 is adapted such that a probe included in sensor 1 is inserted into living body 2 to continuously or semi-continuously measure glucose concentration in blood or an interstitial fluid of living body 2. For example, sensor 1 measures the glucose concentration of living body 2 for several days to several weeks.

FIG 2 is a sectional view of sensor 1. In FIG 2, the same reference signs are provided to the same components as those in FIG 1.

As illustrated in FIG 2, sensor 1 includes main body 11 and probe 12. Probe 12 is inserted into living body 2. Probe 12 includes a reagent layer containing oxidoreductase and outputs an electrical signal based on the glucose concentration to main body 11. Main body 11 stores, in a storage apparatus, the electrical signal based on the glucose concentration output from probe 12 and transmits the electrical signal to another apparatus (not illustrated) at a predetermined timing.

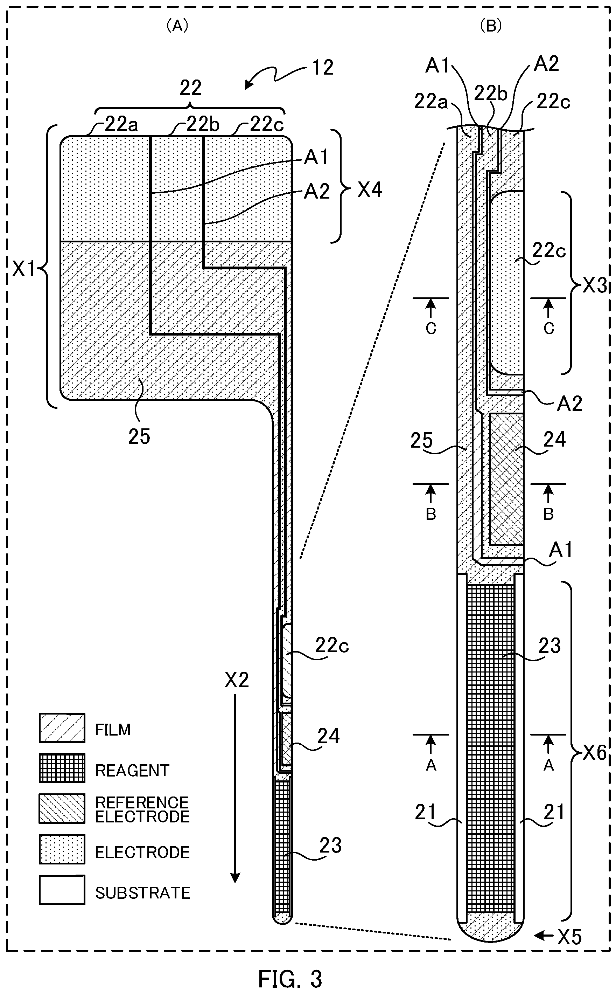

FIG 3 illustrates plan views of probe 12. (A) of FIG 3 illustrates entire probe 12. (B) of FIG 3 illustrates an enlarged view of the distal end part of probe 12 illustrated in (A) of FIG 3.

The part of region X1 (the head portion of probe 12) of probe 12 illustrated in (A) of FIG 3 is accommodated in main body 11. The distal end part of probe 12 projects from main body 11. The distal end part of probe 12 is inserted into living body 2. Arrow X2 illustrated in (A) of FIG 3 indicates an insertion direction of probe 12 into living body 2.

Probe 12 includes substrate 21, electrode 22, reagent layer 23, reference layer 24, and film 25.

A method for manufacturing probe 12 will be schematically described.

### (1) Electrode 22 is formed on substrate 21.

Substrate 21 is, for example, a sheet-shaped synthetic resin. Electrode 22 is uniformly formed on substrate 21.

The material of electrode 22 is, for example, gold (Au). Electrode 22 may be formed on substrate 21 by sputtering, for example. Electrode 22 may be referred to as an electrode film or an electrode layer.

### (2) Electrode 22 is separated into three regions.

Grooves A1 and A2 are formed in electrode 22 formed on substrate 21 to separate electrode 22 into three regions. Electrode 22 is separated into working electrode 22a, reference electrode 22b, and counter electrode 22c by grooves A1 and A2. Grooves A1 and A2 may be formed by laser trimming, for example. Working electrode 22a may be referred to as a working electrode film or a working electrode layer. Reference electrode 22b may be referred to as a reference electrode film or a reference electrode layer. Counter electrode 22c may be referred to as a counter electrode film or a counter electrode layer.

Note that a potential (a potential with reference to the reference electrode) that is sufficient to oxidize a mediator (including not only an electronic mediator but also hydrogen peroxide) reduced by an analyte (glucose) reaction caused by oxidoreductase, for example, is provided to working electrode 22a. The glucose concentration is measured by monitoring a current flowing between working electrode 22a and counter electrode 22c.

### (3) Reference layer 24 is formed.

Reference layer 24 is formed on reference electrode 22b at the distal end part of probe 12. The material of reference layer 24 is, for example, silver/silver chloride (Ag/AgCl). Reference layer 24 may be formed by a screen printing method or an application technique using an Ag/AgCl paste (ink), for example. Reference layer 24 may be referred to as a reference film or a reference electrode.

### (4) Film 25 is disposed and fixed.

Film 25 having an opening is disposed on working electrode 22a, reference electrode 22b, counter electrode 22c, and reference layer 24 formed on substrate 21. Film 25 has a sheet shape and has an insulating property. Film 25 is disposed such that the opening part is located at the distal end part (the part forming reagent layer 23) of probe 12. A reagent, which will be described later, is dropped to the opening of film 25. Film 25 may be referred to as a film layer, an insulating layer, or an insulating film. The disposition may be referred to as lamination or placement instead.

Also, film 25 has an opening such that an upper surface (the surface in the front-side direction of the sheet surface in FIG 3) of counter electrode 22c is partially exposed. The opening of film 25 is cut into a notch shape as illustrated in region X3 in (B) of FIG 3 in the cutting process (7), which will be described later. With the notch shape, a part of counter electrode 22c is exposed in the upper surface. Note that the upper surface may be considered as a surface of probe 12 on a side on which reagent layer 23 is formed.

Also, film 25 has such a shape that the head portion of probe 12 is partially exposed. For example, the part of region X4 in (A) of FIG 3 is not covered with film 25. Exposed electrode 22 in region X4 is connected to a circuit of main body 11.

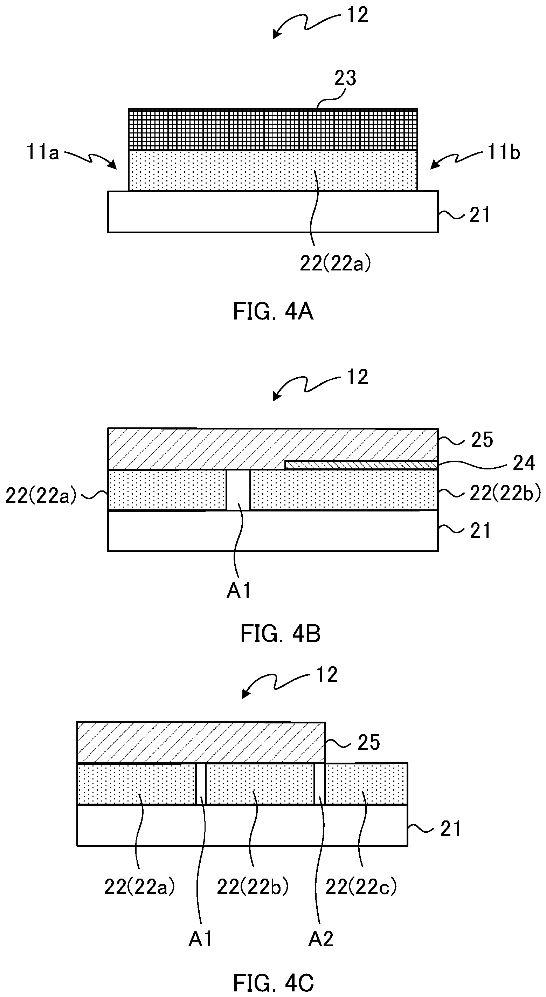

Note that the upper surface of reference layer 24 is covered with film 25 as illustrated in (B) of FIG 3. Reference layer 24 is exposed in the width direction of probe 12 (the direction that is orthogonal to the insertion direction illustrated by arrow X2). In the example in (B) of FIG 3, reference layer 24 is exposed to the right side surface of the distal end part of probe 12 (see reference layer 24 in FIG 4B as well).

### (5) Reagent layer 23 is formed.

Reagent layer 23 is formed on working electrode 22a at the distal end part of probe 12. For example, a reagent is dropped to the opening of film 25, which will be described above, and is then dried to thereby form reagent layer 23. It is preferable that reagent layer 23 be not formed at the distal end of probe 12 illustrated by arrow X5 in (B) of FIG 3. In other words, reagent layer 23 is preferably formed to be separated from the distal end of probe 12. In other words, it is preferable that reagent layer 23 be not formed in a predetermined distance from the distal end of probe 12. This is because it is possible to curb peeling-off (turning-up) of reagent layer 23 from probe 12 when probe 12 is inserted into living body 2 by forming reagent layer 23 to be separated from the distal end of probe 12.

Reagent layer 23 contains at least oxidoreductase capable of causing an oxidation-reduction reaction with the analyte (glucose). Reagent layer 23 may be referred to as a reagent film, a working layer, or a working electrode.

Note that the opening of film 25 may have such a size and a shape that reagent layer 23 with a larger width than the width of probe 12, for example, is formed. Reagent layer 23 formed to have a larger width than that of probe 12 is shaped in the next trimming process.

### (6) Reagent layer 23 and electrode 22 are removed.

Reagent layer 23 and electrode 22 are trimmed along the insertion direction of probe 12 at an end in the width direction of probe 12 with the outer shape formed in the cutting process (7), which will be described later. The upper surface of substrate 21 is partially exposed as illustrated in region X6 in (B) of FIG 3 through the trimming. For the trimming of reagent layer 23 and electrode 22, laser trimming, for example, may be used.

Note that in (B) of FIG 3, film 25 is also partially (a little) trimmed at both end parts of reagent layer 23 in the insertion direction.

### (7) Probe 12 is cut out of substrate 21 through cutting.

Substrate 21 after the above processes (1) to (6) is cut into probe 12 with the shape illustrated in (A) of FIG 3.

The cutting position includes the trimmed part. For example, a part near the center (near the center line) of the trimmed part (the bottom part of the recess) is cut.

### (8) A protective film is formed.

A liquid for forming the protective film, for example, is applied to the distal end part of cut probe 12 to form the protective film. The protective film prevents or curbs leakage of substances (mainly, oxidoreductase and the electron mediator) contained in reagent layer 23 to the outside of the protective film. The protective film has a hole that transmits the analyte that is present outside the protective film into the protective film where reagent layer 23 is present. It is only necessary for the protective film to be able to protect (cover) at least the part corresponding to reagent layer 23 in probe 12.

FIG 4A is a sectional view along arrow AA in FIG 3. As illustrated in FIG 4A, working electrode 22a is formed on (the upper surface of) substrate 21 at the part of probe 12 where reagent layer 23 is formed. Reagent layer 23 is formed on working electrode 22a.

Reagent layer 23 and working electrode 22a are removed at both ends of probe 12 in the width direction (side surfaces of probe 12) in the trimming process (6) described above. In the cutting process (7) described above, substrate 21 exposed in the trimming process (6) described above is cut at a position separated from reagent layer 23 and working electrode 22a. In this manner, the side surfaces of probe 12 has stepped shapes as illustrated by arrows 11a and 11b in FIG 4A.

Note that the protective film is formed in the surroundings of the distal end part of probe 12 at reagent layer 23. In FIG. 4A, illustration of the protective film is omitted.

FIG. 4B is a sectional view along arrow BB in FIG. 3. As illustrated in FIG. 4B, working electrode 22a and reference electrode 22b are formed on substrate 21 at the part of probe 12 where reference layer 24 is formed. Working electrode 22a and reference electrode 22b are physically and electrically separated by groove A1.

Reference layer 24 is disposed on reference electrode 22b. Film 25 is formed on working electrode 22a, reference electrode 22b, and reference layer 24. Reference layer 24 includes the upper surface covered with film 25 and includes a side surface (the right side surface in FIG. 4B) of probe 12 exposed.

FIG. 4C is a sectional view along arrow CC in FIG. 3. As illustrated in FIG. 4C, working electrode 22a, reference electrode 22b, and counter electrode 22c are formed on substrate 21 at the part where the upper surface of counter electrode 22c is exposed. Working electrode 22a and reference electrode 22b are physically and electrically separated by groove A1. Reference electrode 22b and counter electrode 22c are physically and electrically separated by groove A2.

Film 25 is formed on working electrode 22a and reference electrode 22b. Film 25 is not disposed on counter electrode 22c, and the upper surface of counter electrode 22c is exposed.

Examples of each component will be described.

### • Substrate 21

Substrate 21 is a synthetic resin on a sheet. For example, polyethylene terephthalate (PET) may be used for substrate 21. However, the resin material is not particularly limited as long as the resin material has at least one or more features of flexibility, easiness of working, and heat resistance like a plastic material. Other examples include general-purpose plastic such as polyethylene, polypropylene, and polyethylene naphthalate. In a case in which high heat resistance is needed, polyimide is preferably used.

### • Electrode 22

As a material of electrode 22, gold may be used as described above. However, the material is not particularly limited as long as the material is a metal or carbon material with electrical conductivity and stability (for example, it is unlikely to be oxidized or has salinity tolerance). Examples of the material of electrode 22 include platinum, palladium, and carbon.

In a case in which a metal material is used for electrode 22, the metal material may be deposited (including sputtering) on substrate 21. Other formation methods include printing, plating, and spin coating.

In a case in which carbon is used for electrode 22, electrode 22 may be formed by printing a carbon paste. In a case in which one of the upper surface and the back surface of probe 12 is caused to serve as a working electrode and the other surface is caused to serve as a counter electrode, different electrode materials may be used for the working electrode and the counter electrode.

### • Reagent layer 23

Reagent layer 23 contains oxidoreductase capable of causing an oxidation-reduction reaction with at least the analyte as described above. If oxidoreductase is dehydrogenase, an electronic mediator is further contained. Reagent layer 23 may be a system using an electronic mediator even if oxidoreductase is oxidase. In other words, although the electronic mediator is not needed by a system that electrochemically detects hydrogen peroxide generated by the oxidation-reduction reaction of glucose caused by oxidase, electrochemical detection may also be performed using the electronic mediator. In this case, reagent layer 23 includes the electronic mediator in addition to oxidase.

For the system detecting glucose, examples of oxidoreductase include glucose oxidase and glucose dehydrogenase. **In** regard to glucose dehydrogenase, it is desirable to use flavin adenine dinucleotide (FAD)-bound glucose dehydrogenase, and for example, enzymes derived from the genus Aspergillus (oryzae or terreus) or the genus Mucor are preferably used, in terms of low reactivity with respect to maltose.

Examples of the electronic mediator include osmium complexes, ruthenium complexes, quinone compounds, phenazine compounds, and ferrocene compounds. Also, examples of the electronic mediator include derivatives and the like thereof.

### • Reference layer 24

As a material of reference layer 24, silver/silver chloride (Ag/AgCl) may be used as described above. Reference layer 24 may be formed by screen-printing or applying an Ag/AgCl paste (ink) on or to electrode 22 and then drying it.

Note that an example in which sensor 1 according to the present disclosure has a three-electrode configuration of a working electrode, a counter electrode, and a reference electrode for realizing highly accurate measurement is illustrated, sensor 1 may have a two-electrode configuration of a working electrode and a counter electrode.

### • Film 25

As film 25, a product obtained by attaching an adhesive sheet (an acrylic-based, rubber-based, or hot melt-based adhesive sheet, for example) to a sheet of the same material as that of substrate 21 may be used. Also, a sheet of a material that is different from that of substrate 21 may be used. The adhesive sheet may be used alone as film 25. A thermoplastic/photoplastic resist film may be used as film 25.

Film 25 preferably has a contact angle with a liquid on the film that is greater than a contact angle with a liquid at the opening, and a greater difference therebetween is more preferable, in terms of application of the reagent, for example. For example, it is desirable that the contact angle with the liquid on the film be equal to or greater than 90° and the contact angle with the liquid at the opening be equal to or less than 50°. Even if the material does not have such a contact angle, it is also possible to cause the material to have the contact angle by performing at least one of a water repellent treatment on the film surface and a hydrophilic treatment on the opening.

Film 25 has a thickness of equal to or greater than 1 µm and equal to or less than 150 µm, preferably has a thickness of equal to or greater than 3 µm and equal to or less than 50 µm, and more preferably has a thickness of equal to or greater than 5 µm and equal to or less than 30 µm. Film 25 may be formed by printing a resist ink.

### • Protective film

Probe 12 having reagent layer 23 is used by being inserted into living body 2. Therefore, the protective film covering the surface of reagent layer 23 preferably has living body adaptability with which protein and cells are not adsorbed thereto or are unlikely to be adsorbed thereto. In general, the protective film is preferably formed of a polymer having characteristics as described above.

Examples of the polymer include a copolymer of methyl methacrylate and hydroxyethyl methacrylate, a copolymer of butyl methacrylate and hydroxyethyl methacrylate, and poly(2-methacryloyloxyethyl phosphorylcholine-co-n-butyl methacrylate). Note that it is also possible to use a (meth)acrylate-based compound having a similar principal chain to that of these exemplified polymers and having, as a side chain, a reactive group capable of causing a reaction with a linker as an "ethylene-based polymer" having a methacryloyl group or an acryloyl group, which is exemplified as a specific example of a high-molecular-weight polymer.

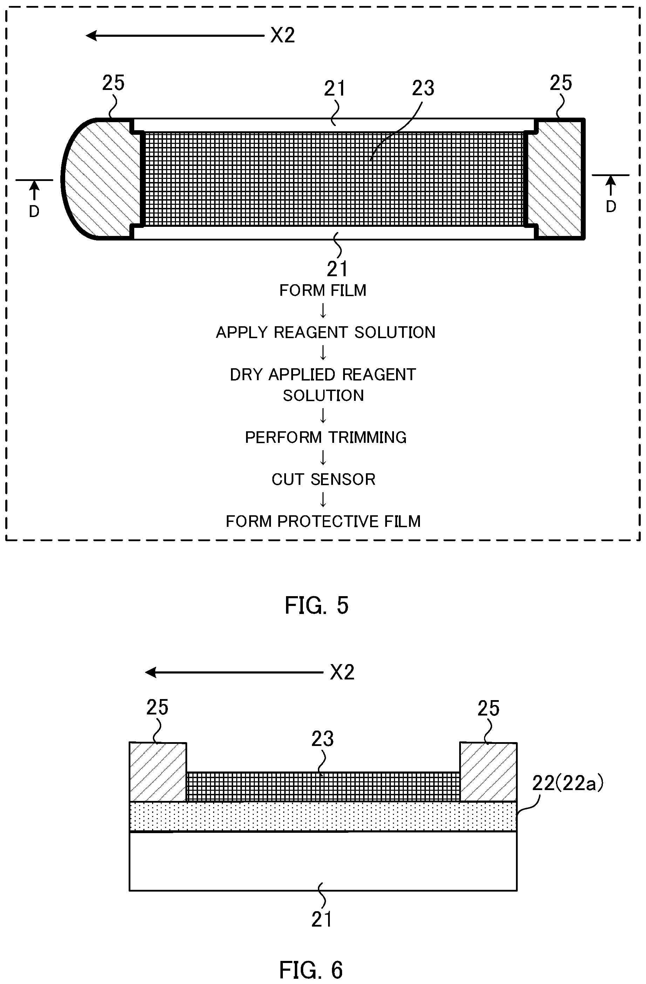

FIG 5 describes a positional relationship between reagent layer 23 and film 25. FIG 5 illustrates a plan view of the distal end part of probe 12. In FIG 5, the same reference signs are provided to the same components as those in FIG 3.

FIG 5 illustrates a part of a process of manufacturing probe 12. "Formation of film" illustrated in FIG 5 corresponds to the aforementioned process (4). "Apply reagent solution" and "dry applied reagent solution" correspond to the aforementioned process (5). "Perform trimming" corresponds to the aforementioned process (6). "Cut sensor" corresponds to the aforementioned process (7). "Form protective film" corresponds to the aforementioned process (8).

Note that in FIG 5, description of the aforementioned processes (1) to (3) is omitted. The process "form film" follows the aforementioned processes (1) to (3). Also, illustration of the protective film is omitted in FIG 5.

FIG 6 is a sectional view along the arrow DD in FIG 5. Film 25 having the opening is disposed on working electrode 22a. The reagent is dropped onto the opening part of film 25 and is then dried. In this manner, reagent layer 23 is formed with reagent layer 23 interposed in film 25 in the insertion direction of probe 12 as illustrated in FIG 6. In other words, reagent layer 23 is formed with reagent layer 23 accommodated in a region defined by the opening of film 25. In other words, film 25 is adjacent to reagent layer 23 on electrode 22.

Note that film 25 on the side of the insertion direction may not be formed. For example, film 25 on the left side illustrated in FIGS. 5 and 6 may be omitted.

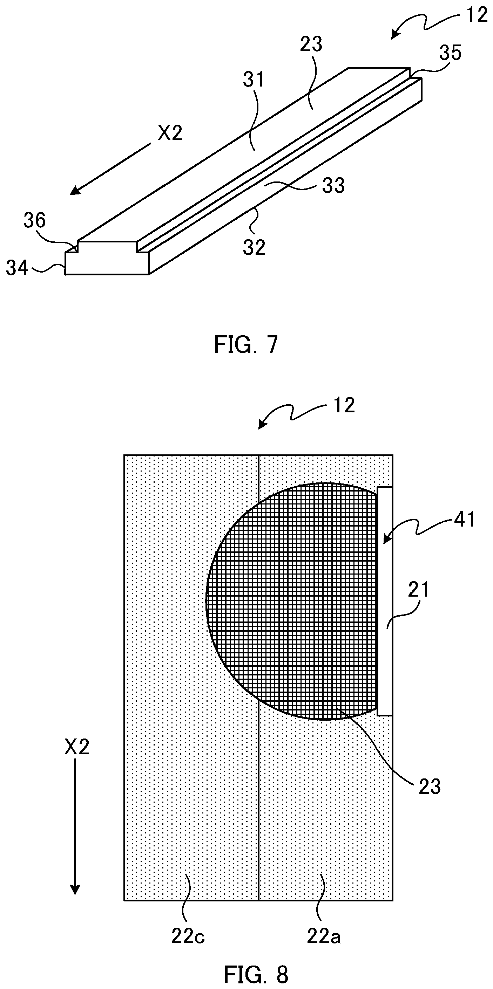

FIG 7 illustrates a perspective view of the part corresponding to reagent layer 23 of probe 12. As illustrated in FIG 7, probe 12 includes upper surface 31 on which reagent layer 23 is formed, back surface 32 that faces upper surface 31, side surface 33 that connects upper surface 31 to back surface 32, and side surface 34 that faces side surface 33 and connects upper surface 31 to back surface 32. Arrow X2 illustrated in FIG 7 indicates the insertion direction of probe 12 into living body 2.

End portions of upper surface 31 of probe 12 in the width direction have stepped trimmed portions 35 and 36 from which reagent layer 23 and electrode 22 have been removed. Trimmed portions 35 and 36 are formed with a positional relationship with which they are in contact at least with reagent layer 23. In other words, reagent layer 23 extends from an end to the other end in the width direction of upper surface 31 to form upper surface 31 of probe 12 and also forms a part of the side surfaces of probe 12 (see reagent layer 23 in FIG 4A as well).

Sensor 1 described above may be regarded as including the following components.

Sensor 1 includes main body 11 and probe 12. Probe 12 is inserted into living body 2 and acquires an electrical signal for continuously or semi-continuously measuring an analyte.

Substrate 21 includes a first surface (for example, upper surface 31) and a second surface (for example, back surface 32) facing the first surface. Also, substrate 21 includes a third surface and a fourth surface (for example, side surfaces 33 and 34) that are surfaces connecting the first surface to the second surface and extending in the insertion direction of probe 12.

Working electrode 22a is formed of a first electrode material on the first surface of substrate 21.

Reagent layer 23 is disposed at a part of working electrode 22a.

Trimmed portions 35 and 36 are formed at both end portions of the first surface in a direction that is orthogonal to the direction along the insertion direction of probe 12 into living body 2 by reagent layer 23 and the first electrode material being removed.

Reagent layer 23 contains oxidoreductase. Trimmed portions 35 and 36 are formed with a positional relationship with which they are in contact at least with reagent layer 23.

Film 25 is adjacent to reagent layer 23 in a direction opposite to the distal end side of probe 12 in the insertion direction into living body 2.

Reagent layer 23 does not have a part interposed between electrode 22 and film 25. In other words, film 25 is not disposed on reagent layer 23. Film 25 may or may not be adjacent to reagent layer 23 on the distal end side of probe 12. In other words, film 25 may or may not be formed on the distal end side of probe 12.

Sensor 1 described above may be regarded as including the following manufacturing process.

First, substrate 21 (substrate sheet) with working electrode 22a of the first electrode material formed on the first surface is prepared.

Next, a reagent solution containing oxidoreductase is applied to a predetermined position on the first surface.

Then, the reagent solution is dried to thereby form reagent layer 23.

Next, the predetermined positions of reagent layer 23 on substrate 21 are trimmed to form trimmed portions, from which reagent layer 23 and working electrode 22a formed below reagent layer 23 have been removed.

Next, substrate 21 is cut into a predetermined shape (the shape of probe 12 illustrated in (A) of FIG. 3, for example, a flagpole shape). The position at which substrate 21 is cut includes trimmed portions 35 and 36.

Note that a protective film may be formed at the distal end part of probe 12 where reagent layer 23 is formed. The protective film includes a hole that can transmit at least an analyte (glucose) therethrough.

Also, counter electrode 22c may be formed on the first surface of substrate 21 or may be formed on the second surface. Another counter electrode (second counter electrode) that is different from the counter electrode 22c may be formed on both or one of the first surface and the second surface of substrate 21.

Also, reference electrode 22b may be formed on at least one of the first to fourth surfaces. In a case in which reference layer 24 is formed on the first surface, film 25 is disposed on the upper surface with the third surface side exposed.

Also, reagent layer 23 may not be formed in a predetermined distance from the terminal end side (the distal end of probe 12) of the first surface in the insertion direction of probe 12.

A part (end portion) of reagent layer 23 may be interposed between electrode 22 and film 25. Reagent layer 23 may not have a part interposed between electrode 22 and film 25.

As described above, sensor 1 includes probe 12 that is to be inserted into living body 2 to measure an analyte. Probe 12 includes substrate 21, electrode 22 that is formed on substrate 21, and reagent layer 23 that contains oxidoreductase and is formed on electrode 22. Reagent layer 23 and electrode 22 of probe 12 are trimmed at least one of end portions in the width direction along the insertion direction of probe 12 into living body 2.

In this manner, variations of performance of sensor 1 caused by the manufacturing process are reduced. For example, even if a so-called coffee ring state in which reagent layer 23 dropped onto electrode 22 is thicker at its edge part than at its center portion is achieved, it is possible to use a uniform (even) part inside the ring as reagent layer 23 by the trimming.

Also, it is possible to prevent a blade tip from coming into contact with reagent layer 23 at the time of the cutting into the shape of probe 12 and to reduce cracking of reagent layer 23.

Also, it is possible to prevent the blade tip from coming into contact with reagent layer 23 at the time of the cutting into the shape of probe 12 and to reduce contamination of the reagent.

As described above, probe 12 included in sensor 1 is manufactured by a process of forming electrode 22 on substrate 21, a process of forming reagent layer 23 containing oxidoreductase on electrode 22, and a process of trimming reagent layer 23 and electrode 22 from at least one of end portions of probe 12 in the width direction along the insertion direction of probe 12 into living body 2.

In this manner, variations in performance of sensor 1 caused by the manufacturing process are reduced. For example, even if reagent layer 23 dropped onto electrode 22 is brought into the coffee ring state, it is possible to use a uniform (even) center part inside the ring as reagent layer 23 by the trimming.

It is possible to prevent the blade tip from coming into contact with reagent layer 23 by the trimming at the time of the cutting into the shape of probe 12 and thereby to reduce cracking of reagent layer 23. Also, it is possible to reduce contamination of the reagent.

As described above, film 25 is disposed on electrode 22 such that it is adjacent to reagent layer 23 at both end portions of reagent layer 23 in the insertion direction.

In this manner, variations in performance of sensor 1 caused by the manufacturing process are reduced. For example, it is possible to determine, by film 25, the position at which the reagent is to be dropped and to form uniform reagent layer 23 before the trimming.

### (Modification Example 1)

Probe 12 may have a trimmed portion at one of the ends in the width direction. In other words, there may be one trimmed portion.

FIG 8 illustrates a plan view of the distal end part of probe 12. In FIG 8, the same reference signs are provided to the same components as those in FIG 3. FIG 8 illustrates an example in which working electrode 22a and counter electrode 22c are formed in an aligned manner in the width direction of probe 12. In FIG 8, illustration of film 25 is omitted.

Reagent layer 23 is formed to cross over the width of probe 12 on one end side of probe 12 in the width direction. Reagent layer 23 is formed not to cross over the width of probe 12 on the other end side of probe 12 in the width direction. In the example of FIG 8, reagent layer 23 is formed to cross over the right end of probe 12 and is formed not to cross over the left end of probe 12, for example.

Probe 12 includes trimmed portion 41. Trimmed portion 41 is formed on the side on which reagent layer 23 crosses over the width of the probe (the right side in FIG 6). Trimmed portion 41 is formed by trimming reagent layer 23 and working electrode 22a. Substrate 21 is exposed by trimmed portion 41.

In this manner, probe 12 may have the trimmed portion at one of the ends in the width direction. This also leads to reduction of variations in performance of sensor 1 caused by the manufacturing process.

### (Modification Example 2)

Reagent layer 23 may stick out of the region defined by film 25 in the insertion direction of probe 12.

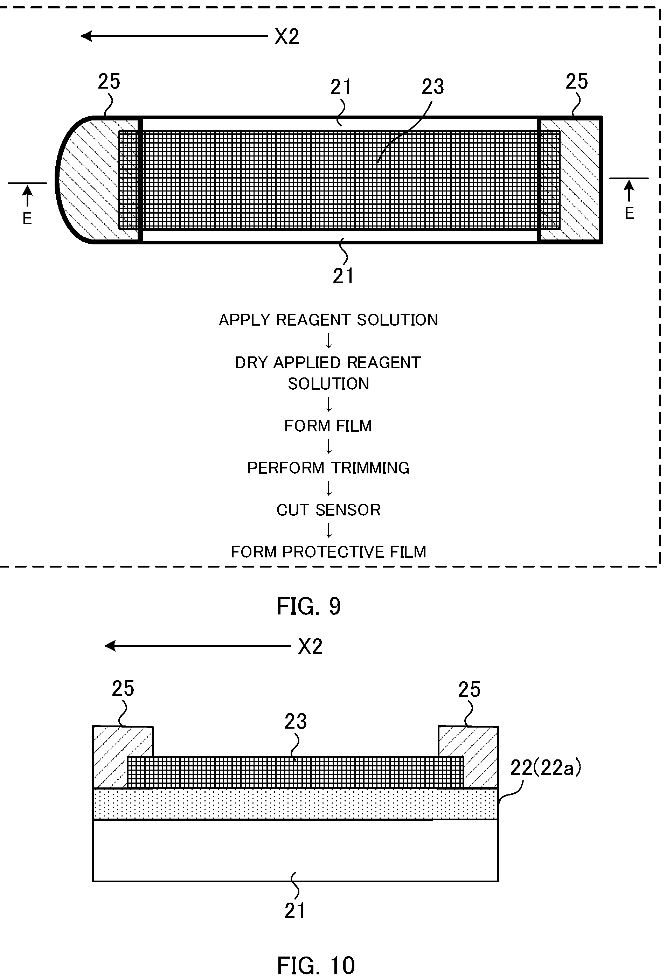

FIG 9 is a diagram for describing a positional relationship between reagent layer 23 and film 25. FIG 9 illustrates a plan view of the distal end part of probe 12. In FIG 9, the same reference signs are provided to the same components as those in FIG 3.

FIG 9 illustrates a part of the process of manufacturing probe 12. "Apply reagent fluid" and "dry applied reagent" illustrated in FIG 9 correspond to the aforementioned process (5). "Form film" corresponds to the aforementioned process (4). "Perform trimming" corresponds to the aforementioned process (6). "Cut sensor" corresponds to the aforementioned process (7). "Form protective film" corresponds to the aforementioned process (8).

Note that in FIG 9, description of the aforementioned processes (1) to (3) is omitted. The process "apply reagent solution" illustrated in FIG 9 follows the aforementioned processes (1) to (3). Also, illustration of the protective film is omitted in FIG 9.

FIG 10 illustrates a sectional view along arrow EE in FIG 9. Film 25 having an opening is disposed on reagent layer 23. Film 25 is disposed such that the opening part is located at reagent layer 23. The opening of film 25 is formed to overlap reagent layer 23 at both ends of reagent layer 23 in the insertion direction (the direction of arrow X2). In other words, a part of film 25 overlaps reagent layer 23 at both ends of reagent layer 23 in the insertion direction. Also, a part of film 25 overlaps the trimmed portions at both ends of the trimmed portions in the insertion direction.

Note that film 25 on the side of the insertion direction may not be formed. For example, film 25 on the left side illustrated in FIGS. 9 and 10 may be omitted.

In this manner, film 25 is disposed on electrode 22 to overlap reagent layer 23 and trimmed portions at both end portions of reagent layer 23 in the insertion direction.

In this manner, variations in performance of sensor 1 caused by the manufacturing process are reduced. For example, it is possible to cover the end portions (the edge parts of the coffee ring) of reagent layer 23 in the insertion direction of probe 12 with film 25 and expose the uniform part of reagent layer 23.

### (Modification Example 3)

Examples of the opening shape of film 25 will be described.

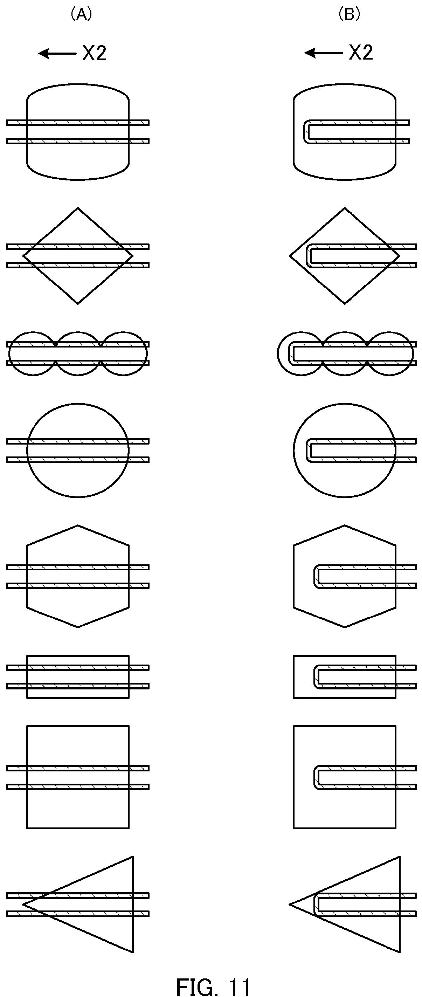

FIG 11 illustrates examples of the opening shape of film 25. The hatched parts in (A) of FIG 11 and (B) of FIG 11 illustrate trimmed portions. The figures with polygonal shapes, circular shapes, and the like illustrated in (A) of FIG 11 and(B) of FIG 11 illustrate the shapes of the opening part of film 25. Arrow X2 illustrated in FIG 11 indicates the insertion direction of probe 12 into living body 2.

In (A) of FIG 11, film 25 is formed at both ends of reagent layer 23 in the insertion direction (see FIGS. 5 and 6, for example). In (B) of FIG 11, film 25 is formed at an end on a side opposite to the distal end side of reagent layer 23 (film 25 is formed on the right side in FIGS. 5 and 6 and film 25 on the left side is not formed, for example). In this manner, the opening shape of film 25 may be various shapes.

### (Modification Example 4)

An example of the size of sensor 1 will be described.

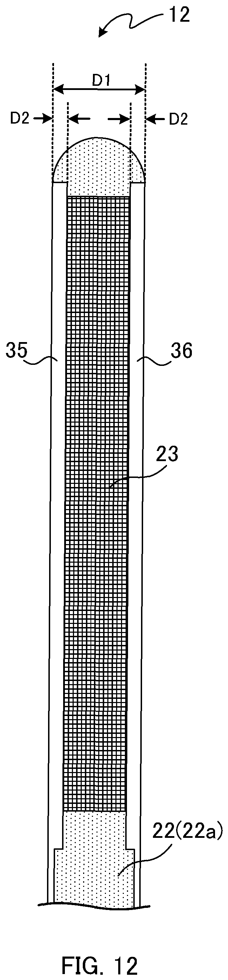

FIG 12 describes an example of the size of sensor 1. In FIG 12, the same reference signs are provided to the same components as those in FIGS. 3 and 7. In FIG 12, illustration of film 25 is omitted.

Width D1 of distal end part of probe 12 is, for example, equal to or greater than 70 µm and equal to or less than 1700 µm. Width D1 is preferably equal to or greater than 70 µm and equal to or less than 600 µm and is more preferably equal to or greater than 70 µm and equal to or less than 400 µm.

Width D2 of trimmed portions 35 and 36 is, for example, equal to or greater than 5 µm. Width D2 is not particularly limited as long as a condition of a width with which it is possible to secure reagent layer 23 with respect to the width of the distal end part of probe 12 is met. In a case in which it is desired to widen width D2 of trimmed portions 35 and 36, it can be realized by performing irradiation with a laser a plurality of times.

### (Embodiment 2)

For example, an electrochemical sensor called a CGM sensor continuously or semi-continuously measures an analyte in a living body for several days to several weeks. At that time, there may be a case in which signal intensity obtained from a probe differs even if the concentration of the analyte is the same depending on a measurement timing such as a day or a time, and measurement accuracy of the sensor may be degraded.

The present inventors have considered that one of the reasons is that Ag/AgCl (silver or silver chloride) that is a material of a reference layer is decomposed due to long-time continuous power distribution to the reference layer and decomposed Ag/AgCl flows into the working electrode side. Also, the present inventors have presumed that the reason that Ag/AgCl flowing into the working electrode side causes abnormality of a signal value is based on a reaction of silver ions or chloride ions with a reagent on the working electrode side. Therefore, it is possible to curb the degradation of measurement accuracy of the sensor by curbing decomposition of Ag/AgCl in the reference layer.

One of means for curbing the decomposition of Ag/AgCl in the reference layer is miniaturizing the reference layer and reducing an area in which the reference layer comes into contact with the analyte. Examples of techniques for constructing the Ag/AgCl reference layer include a screen printing technique and an application technique.

However, in a case in which a minute electrode is formed by the screen printing technique, clogging of a printing plate is likely to occur due to the minute electrode. Also, the screen printing technique causes a large loss of the Ag/AgCl paste. Therefore, the screen printing technique is not suitable in terms of mass production of sensors.

Although a plating technique is a suitable technique in terms of miniaturization of the Ag/AgCl electrode (reference layer), the plating technique is not preferable for mass production of sensors. A first reason is that management of chemicals used for the plating and a manufacturing process is complicated. Second, it is difficult to mask a probe part other than the minute Ag/AgCl electrode when the plating of the Ag/AgCl electrode is performed and to continuously perform the management.

Hereinafter, a sensor capable of reducing the area in which the reference layer comes into contact with the analyte and measuring the analyte with higher accuracy and a method for manufacturing the same will be described.

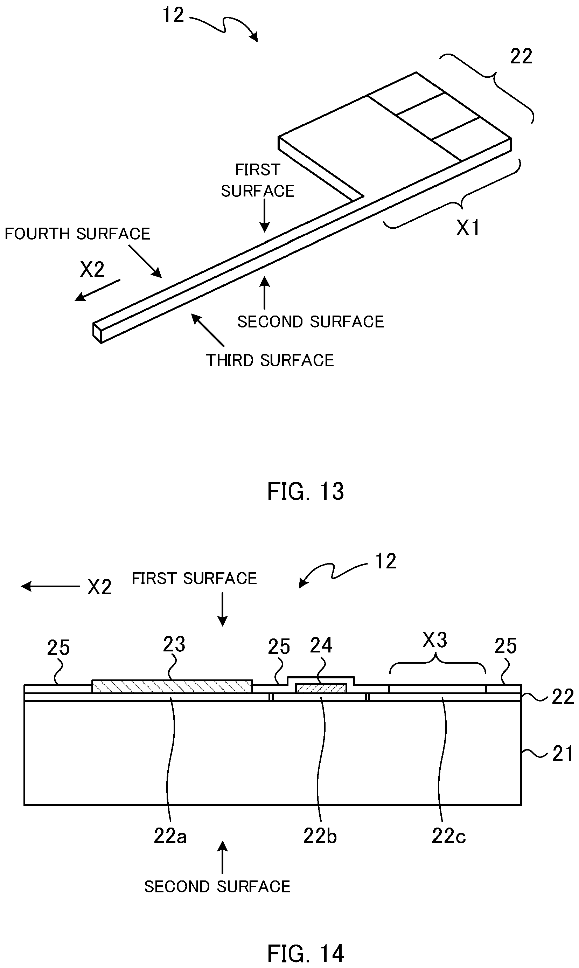

FIG 13 illustrates a perspective view of probe 12 of sensor 1 according to Embodiment 2. In FIG 13, the same reference signs are provided to the same components as those in FIGS. 2 and 3.

Probe 12 illustrated in FIG 13 includes electrode 22 similarly to probe 12 described in Embodiment 1. Probe 12 includes reagent layer 23 (not illustrated in FIG 13), reference layer 24 (not illustrated in FIG 13), and counter electrode 22c, a part of which is exposed in a first surface (not illustrated in FIG 13) similarly to probe 12 described in Embodiment 1.

The surface of probe 12 on the side on which reagent layer 23 and reference layer 24 are formed may be referred to as a first surface. The surface facing the first surface may be referred to as a second surface.

Also, a surface that connects the first surface to the second surface and extends in the insertion direction (the direction of arrow X2 in FIG 13) of probe 12, which is the right side surface when seen from the distal end of probe 12, may be referred to as a third surface. A surface that connects the first surface to the second surface and extends in the insertion direction of probe 12, which is the left side surface when seen from the distal end of probe 12, may be referred to as a fourth surface.

Note that the first surface may also be referred to as an upper surface. The second surface may also be referred to as a bottom surface. The third surface and the fourth surface may also be referred to as side surfaces.

FIG 14 illustrates a partial side view of probe 12 in FIG 13 when seen from the third surface side. As illustrated in FIG 14, electrode 22 is formed on the first surface side of substrate 21. Electrode 22 includes working electrode 22a, reference electrode 22b, and counter electrode 22c.

Reagent layer 23 is formed on working electrode 22a of electrode 22 on the first surface side. Reference layer 24 is formed on reference electrode 22b of electrode 22 on the first surface side. Reference layer 24 is formed by hardening an Ag/AgCl paste applied to reference electrode 22b.

Reference layer 24 on the side of the first surface is covered with film 25. In other words, reference layer 24 on the first surface side is not exposed. Reference layer 24 is exposed on the third surface side of probe 12. In other words, reference layer 24 is exposed in the width direction (the direction that is orthogonal to the insertion direction illustrated by arrow X2) of probe 12 (see FIG. 4B as well).

Note that reference layer 24 exposed on the third surface side may be covered with a protective film as described in Embodiment 1. As described above in Embodiment 1, the protective film has a hole through which at least the analyte (glucose) can be transmitted. Therefore, reference layer 24 is exposed to (contacts with, distributes power to) at least the analyte.

Film 25 has an opening such that the part in region X3 of counter electrode 22c on the first surface side is exposed. The opening of film 25 has a notch shape (see region X3 in (B) of FIG. 3 as well). Counter electrode 22c is partially exposed in the upper surface by the notch shape.

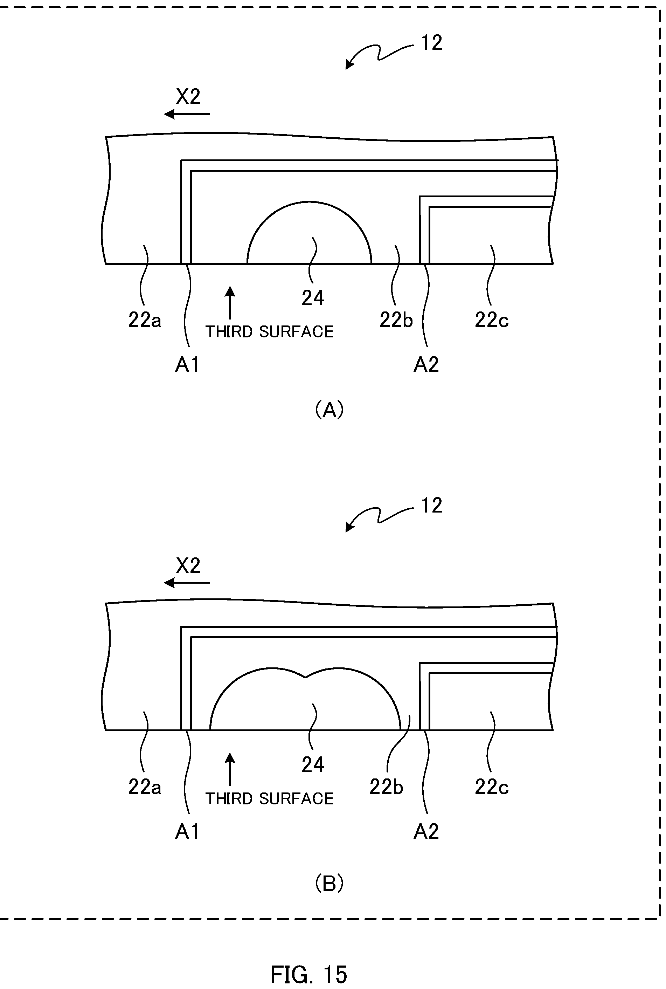

The shape of reference layer 24 in the direction from the third surface toward the fourth surface when seen from the first surface may be an arc shape, an elliptical arc shape, or the like (see FIG. 15, for example).

Note that the part corresponding to working electrode 22a from the head portion (the part in region X1 in FIG. 13) of probe 12 to reagent layer 23 may be referred to as a lead or a working electrode lead. The part corresponding to reference electrode 22b from the head portion of probe 12 to reference layer 24 may be referred to as a lead or a reference electrode lead. The part corresponding to counter electrode 22c from the head portion of probe 12 to the opening (region X3) of film 25 may be referred to as a lead or a counter electrode lead.

Also, reference layer 24 is exposed on the fourth surface side. Reference layer 24 and counter electrode 22c may be formed on the second surface side. In the case in which reference layer 24 and counter electrode 22c are formed on the second surface side, the reference electrode lead and the counter electrode lead are formed on the second surface side.

FIG. 15 describes shape examples of reference layer 24. (A) of FIG. 15 and (B) of FIG 15 illustrate reference layer 24 when probe 12 is seen from the first surface side. In (A) of FIG 15 and (B) of FIG 15, illustration of film 25 is omitted. (A) of FIG 15 and (B) of FIG 15 illustrate grooves A1 and A2 that separate working electrode 22a, reference electrode 22b, and counter electrode 22c of electrode 22 (see (B) of FIG 3 as well).

As illustrated in (A) of FIG 15, the shape of reference layer 24 in the direction from the third surface toward the fourth surface when probe 12 is seen from the first surface side may be an arc shape. Also, the shape of reference layer 24 from the third surface toward the fourth surface when probe 12 is seen from the first surface side may be an elliptical arc shape. In other words, the shape of reference layer 24 may be a shape narrowed in the direction from the exposed surface (third surface) of reference layer 24 toward the center portion (inside) of reference layer 24 when seen from the first surface side.

Reference layer 24 may have a shape of a plurality of continuous arcs or elliptical arcs. As illustrated in (B) of FIG 15, for example, reference layer 24 may have a shape of two continuous arcs. In other words, reference layer 24 may have a first arc and a second arc or a first elliptical arc and a second elliptical arc. Alternatively, reference layer 24 may have a shape of a combination of an arc and an elliptical arc.

As illustrated in (A) of FIG 15 and(B) of FIG 15, reference electrode 22b (reference electrode lead) has a width (area) widened on the distal end side of probe 12. The Ag/AgCl paste is applied to a part of the region where the width of the reference electrode lead is widened.

An outline of a method for manufacturing probe 12 (reference layer 24 of probe 12) will be described.

Process 1: Electrode 22 is formed on the first surface side of sheet-shaped substrate 21.

Process 2: The Ag/AgCl paste is applied to a predetermined position on electrode 22.

Process 3: The applied Ag/AgCl paste is dried.

Process 4: Film 25 is formed at a part including the dried Ag/AgCl.

Process 5: Sheet-shaped substrate 21 is cut into a probe shape. When cutting is performed, the cutting is performed such that a part of dried Ag/AgCl is included.

Note that next Process 1a may follow Process 1. Also, next Process 2a may be performed instead of Process 2.

Process 1a: Grooves A1 and A2 are formed in electrode 22 formed on substrate 21 to form working electrode 22a, reference electrode 22b, and counter electrode 22c.

Process 2a: The Ag/AgCl paste is applied to a predetermined position on reference electrode 22b formed in Process 1a. For example, the Ag/AgCl paste is applied to a part of a region of reference electrode 22b (reference electrode lead) where the width is widened on the distal end side of probe 12.

Also, Process 2 may be performed as following Process 2b.

Process 2b: When the Ag/AgCl paste is applied to the first surface side of sheet-shaped substrate 21, the application is performed such that a part of Ag/AgCl includes the part corresponding to reference electrode 22b (reference electrode lead). In other words, the Ag/AgCl paste is applied to cross over the part at which substrate 21 is cut in the cutting in Process 5.

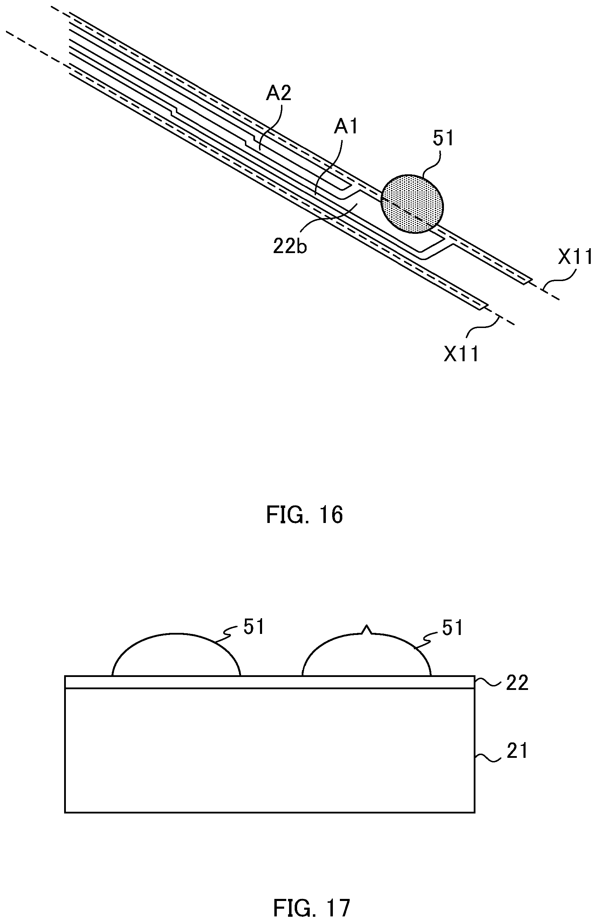

FIG 16 illustrates an application example of Ag/AgCl paste 51. FIG 16 illustrates electrode 22 formed on substrate 21 (the reference sign is omitted in FIG 16), grooves A1 and A2, and Ag/AgCl paste 51. Dashed line X11 illustrated in FIG 16 illustrates a part cut in Process 5. In FIG 16 illustration of the head portion and the distal end portion of probe 12, reagent layer 23, and film 25 is omitted.

Ag/AgCl paste 51 is applied to the end portion (the part where the width of reference electrode 22b is widened) of reference electrode 22b in the insertion direction of probe 12 as illustrated in FIG 16. Also, Ag/AgCl paste 51 is applied to cross over the part (dashed line X11) at which substrate 21 is cut in the cutting in Process 5. Therefore, substrate 21 is cut to cross over the part to which Ag/AgCl paste 51 is applied in Process 5.

Note that film 25 is formed on the upper surface of Ag/AgCl paste 51 in Process 4. Therefore, reference layer 24 is not exposed from the upper surface (first surface) of probe 12 but is exposed from the side surface (third surface) as described in FIG 14.

FIG 17 illustrates a side view after Ag/AgCl paste 51 is applied. FIG 17 illustrates substrate 21, electrode 22, and Ag/AgCl paste 51.

The application technique is suitable for forming minute electrode (reference layer 24 of Ag/AgCl). However, in regard to the surface of reference layer 24 formed by the application technique, no flat plane is present on the surface of reference layer 24, and the surface area thereof is large, as is understood from the side view in FIG 17. Also, Ag/AgCl paste 51 may have a protrusion (raised part) on the surface as illustrated in Ag/AgCl paste 51 on the right side (note that the protrusions may be eliminated by the cutting in Process 5).

On the other hand, Ag/AgCl paste 51 is applied to reference electrode 22b such that a part of Ag/AgCl paste 51 crosses over the cut part of substrate 21 in this case. Also, the upper surface of applied Ag/AgCl paste 51 is covered with film 25, and substrate 21 is cut. It is thus possible to reduce the section (exposed part) area of reference layer 24.

Also, variations in surface area of Ag/AgCl paste 51 are likely to become large in the application method.

On the other hand, Ag/AgCl paste 51 is applied to reference electrode 22b such that a part of Ag/AgCl paste 51 crosses over the cut part of substrate 21 in this case. Also, the upper surface of applied Ag/AgCl paste 51 is covered with film 25, and substrate 21 is cut. It is thus possible to curb variations in the section (exposed part) area of reference layer 24.

In this manner, sensor 1 can measure the analyte with high accuracy. Also, mass production of sensor 1 can be easily achieved.

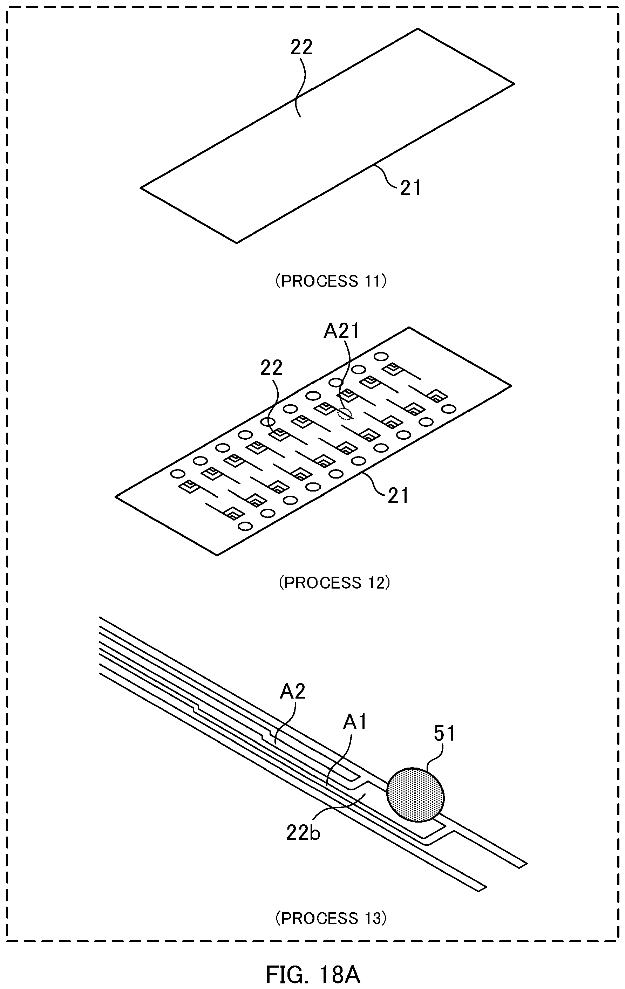

A detailed method for manufacturing probe 12 will be described. FIGS. 18A to 18C describe an example of the method for manufacturing probe 12.

### (Process 11)

As illustrated in (Process 11) in FIG 18A, electrode 22 is formed on sheet-shaped substrate 21. For example, an electrode material such as gold is sputtered on sheet-shaped substrate 21 of polyethylene terephthalate (PET), for example, to form electrode 22.

### (Process 12)

As illustrated in (Process 12) in FIG 18A, electrode 22 is trimmed to form working electrode 22a, reference electrode 22b, and counter electrode 22c (the reference signs are omitted in (Process 12) in FIG 18A). For the trimming of electrode 22, laser trimming may be used, for example.

### (Process 13)

As illustrated in (Process 13) in FIG 18A, Ag/AgCl paste 51 is applied to reference electrode 22b to cross over the cut part (cut line) of substrate 21 and is then dried. In other words, Ag/AgCl paste 51 is applied to cross over reference electrode 22b and a part that eventually corresponds to the outside of probe 12 after the cutting and is then dried. In this manner, reference layer 24 is formed on electrode 22 (reference electrode 22b).

Note that (Process 13) in FIG 18A is an enlarged view of the part corresponding to dashed line frame A21 in (Process 12) in FIG 18A and is the same diagram as FIG 16. (Process 13) in FIG 18A illustrates reference electrode 22b formed on substrate 21, grooves A1 and A2, and Ag/AgCl paste 51. In (Process 13) in FIG 18A, illustration of the head portion and the distal end portion of probe 12, reagent layer 23, and film 25 is omitted.

### (Process 14)

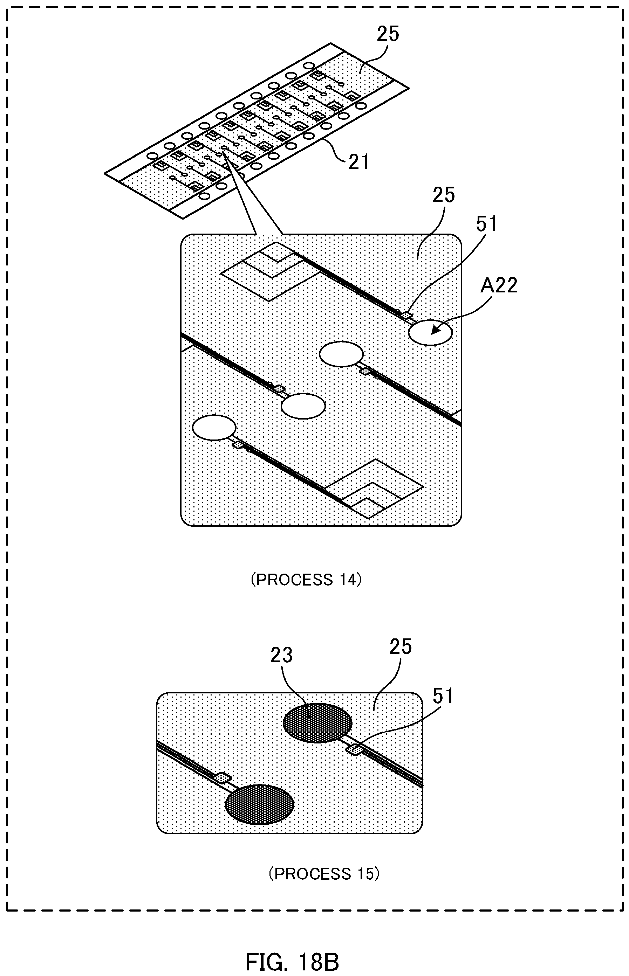

As illustrated in (Process 14) in FIG 18B, film 25 in which the part where reagent layer 23 of working electrode 22a is to be formed and the part corresponding to region X3 of counter electrode 22c are opened is attached to substrate 21. At this time, reference electrode 22b (reference layer 24) on the first surface side is covered with film 25 (see FIG 14).

Note that arrow A22 in (Process 14) in FIG 18B illustrates the opening in film 25 at the part where reagent layer 23 is to be formed. Illustration of the opening in film 25 at the part corresponding to region X3 (see FIG 14) of counter electrode 22c is omitted.

### (Process 15)

As illustrated in (Process 15) in FIG 18B, a reagent solution is applied to the opening part (the part of arrow A22 illustrated in (Process 14) in FIG 18B) in film 25 for forming reagent layer 23 and is then dried to form reagent layer 23.

### (Process 16)

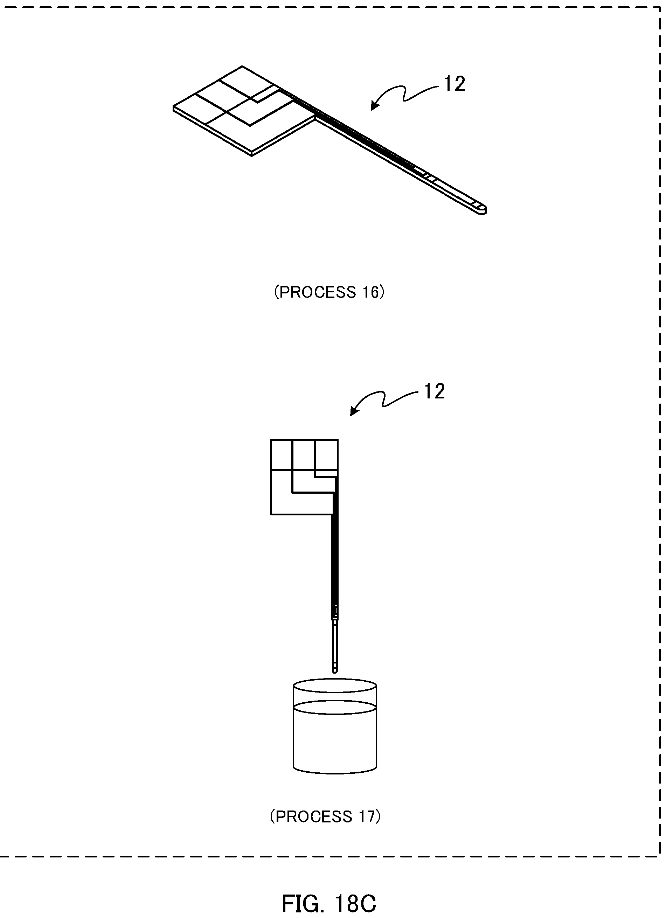

As illustrated in (Process 16) in FIG 18C, substrate 21 is cut into the shape of probe 12 to include a part of reagent layer 23 and a part of Ag/AgCl paste 51. Reference layer 24 is covered with film 25 on the first surface side of probe 12 but is exposed on the third surface side.

### (Process 17)

As illustrated in (Process 17) in FIG 18C, the part corresponding at least to reagent layer 23 of probe 12 is covered with a protective film through dipping, for example.

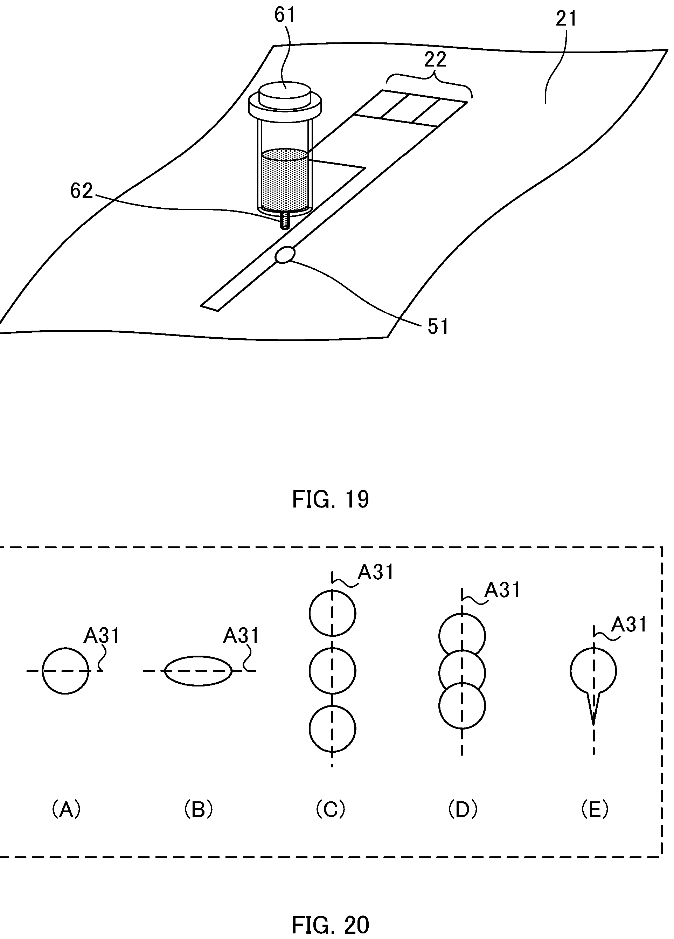

FIG 19 describes the application of Ag/AgCl paste 51. FIG 19 illustrates a part of substrate 21 with electrode 22 formed thereon and application apparatus 61. Application apparatus 61 includes nozzle 62. Ag/AgCl paste 51 is ejected from nozzle 62 and is applied to reference electrode 22b.

FIG 20 describes the shape after Ag/AgCl paste 51 is applied. As illustrated in (A) to (D) in FIG 20, Ag/AgCl paste 51 applied to substrate 21 may have a circular shape or an elliptical shape. As illustrated in (E) in FIG 20, Ag/AgCl paste 51 may have a speech balloon shape. The speech balloon shape is formed by a squeegee technique, which will be described later, for example.

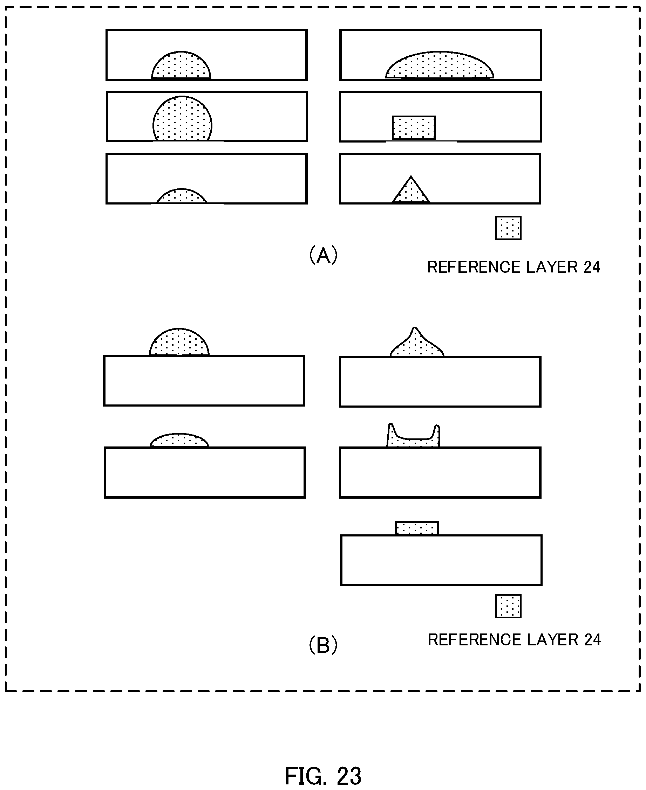

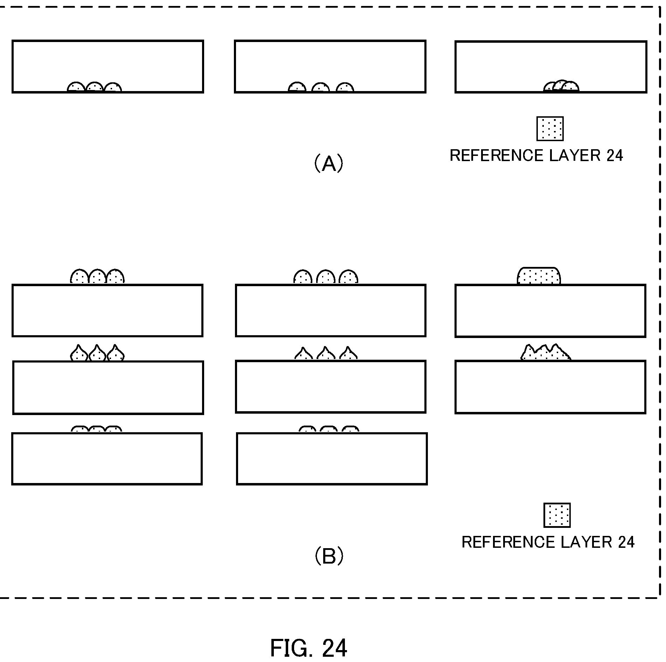

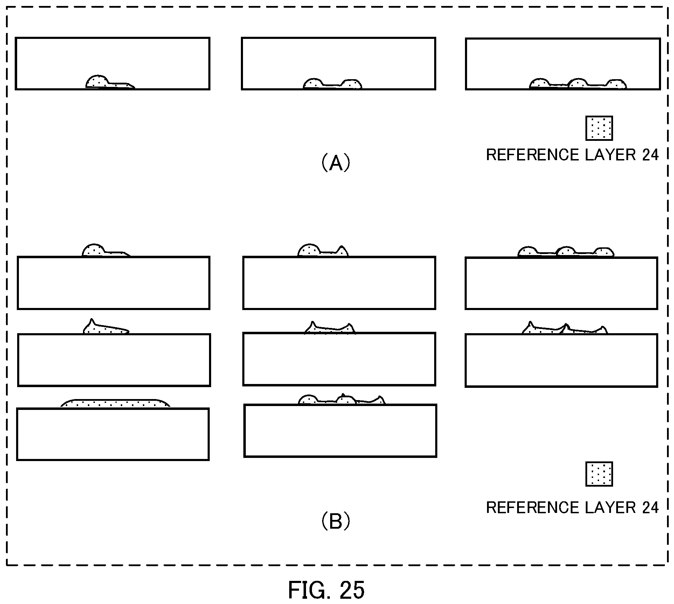

Dashed line A31 illustrated in FIG 20 illustrates a cut line of the cutting performed in Process 16 described above, for example. Therefore, the outer circumferential shape of reference layer 24 when seen from the upper surface side is an arc shape, an elliptical arc shape, a shape obtained by cutting the speech balloon shape into a half, or the like due to the cutting of substrate 21 (see (A) in FIG 23, (A) in FIG 24, and (A) in FIG 25, for example).

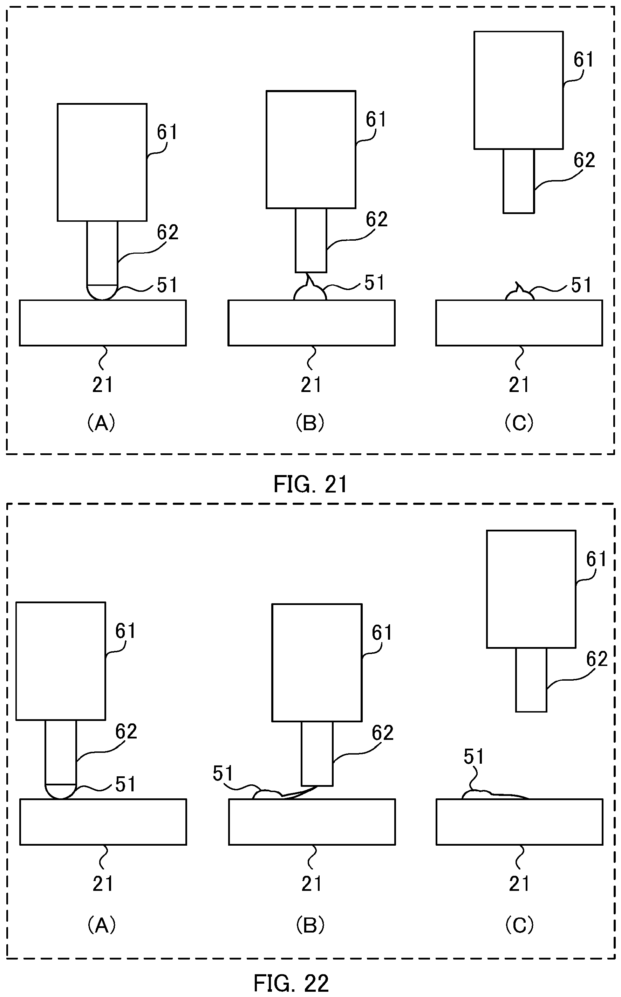

FIG 21 describes operations of application apparatus 61. As illustrated in (A) in FIG 21, application apparatus 61 is moved to the vicinity of substrate 21. Then, application apparatus 61 ejects Ag/AgCl paste 51 from nozzle 62.

Application apparatus 61 moves upward as illustrated (B) in FIG 21 and (C) in FIG 21 after ejecting Ag/AgCl paste 51.

In this manner, Ag/AgCl paste 51 is applied to reference electrode 22b on substrate 21.

Note that in a case in which application apparatus 61 performs the operations as illustrated in FIG 21 once on reference electrode 22b on substrate 21, one piece of Ag/AgCl paste 51 is applied to reference electrode 22b on substrate 21 (see (A) and (B) in FIG 20, for example). The technique of applying one piece of Ag/AgCl paste 51 to reference electrode 22b on substrate 21 may be referred to as a single-application technique.

Also, in a case in which application apparatus 61 performs the operations illustrated in FIG 21 twice or more on reference electrode 22b on substrate 21, two or more pieces of Ag/AgCl paste 51 are applied to reference electrode 22b on substrate 21 (see (C) and (D) in FIG 20, for example). The technique of applying two or more pieces of Ag/AgCl paste 51 to reference electrode 22b on substrate 21 may be referred to as a repeated-application technique.

FIG 22 describes operations of application apparatus 61. As illustrated in (A) in FIG 22, application apparatus 61 is moved to the vicinity of substrate 21. Then, application apparatus 61 ejects Ag/AgCl paste 51 from nozzle 62.

Next, application apparatus 61 moves in parallel while maintaining the distance from substrate 21 as illustrated in (B) in FIG. 22.

Then, application apparatus 61 moves upward as illustrated in (C) in FIG 22.

In this manner, Ag/AgCl paste 51 is applied to form a speech balloon shape on reference electrode 22b on substrate 21 as illustrated in (E) in FIG 20.

Note that the technique of moving application apparatus 61 in parallel while the distance from substrate 21 is maintained and applying Ag/AgCl paste 51 to substrate 21 may be referred to as a squeegee technique.

FIG 23 illustrates shape examples of reference layer 24 obtained by a single-application technique. (A) in FIG 23 illustrates shape examples of reference layer 24 when seen from the first surface side of probe 12 after the cutting of substrate 21. (B) in FIG 23 illustrates shape examples of reference layer 24 when seen from the third surface side of probe 12 after the cutting of substrate 21.

Reference layer 24 may be formed into an arc shape when seen from the first surface side of probe 12 as illustrated in (A) in FIG 23. For example, reference layer 24 may have an arc shape (semicircular shape) with a center angle of 180°. Also, reference layer 24 may have an arc shape with a center angle of greater than 180°. Reference layer 24 may have an arc shape with a center angle of less than 180°. Reference layer 24 may be formed into an elliptical arc shape, a quadrangular shape, or a triangular shape.

Reference layer 24 may be formed into an arc shape, an elliptical arc shape, an arc shape or an elliptical arc shape with a protrusion at a center portion, a shape with protrusions at a periphery thereof, or a quadrangular shape when seen from the third surface side of probe 12 as illustrated in (B) in FIG 23.

As the shape of reference layer 24, various shapes can be set depending on the shape of nozzle 62 of application apparatus 61. However, the shape having protrusions at a periphery thereof in (B) in FIG 23 is formed by a spot application scheme rather than the nozzle shape.

FIG 24 illustrates shape examples of reference layer 24 obtained by the repeated-application technique. (A) in FIG 24 illustrates shape examples of reference layer 24 when seen from the first surface side of probe 12 after the cutting of substrate 21. (B) of FIG 24 illustrates shape examples of reference layer 24 when seen from the third surface side of probe 12 after the cutting of substrate 21.

Reference layers 24 may be formed into arc shapes when seen from the first surface side of probe 12 as illustrated in (A) in FIG 24. Reference layers 24 may be adjacent to each other or may be separated from each other. Reference layers 24 may overlap one another. Although not illustrated in (A) in FIG 24, the shapes of reference layers 24 are not limited to the arc shapes and may be elliptical arc shapes, quadrangular shapes, or triangular shapes.

Reference layers 24 may be formed into arc shapes, elliptical arc shapes, arc shapes or elliptical arc shapes with protrusions, shapes with protrusions at the center portions, or quadrangular shapes when seen from the third surface side of probe 12 as illustrated in (B) in FIG 24.

As the shapes of reference layers 24, various shapes can be set depending on the shape of nozzle 62 of application apparatus 61.

FIG 25 illustrates shape examples of reference layer 24 obtained by the squeegee technique. (A) in FIG 25 illustrates shape examples of reference layer 24 when seen from the first surface side of probe 12 after the cutting of substrate 21. (B) in FIG 25 illustrates shape examples of reference layer 24 when seen from the third surface side of probe 12 after the cutting of substrate 21.

Reference layer 24 may be formed into a shape of a half speech balloon when seen from the first surface side of probe 12 as illustrated in (A) in FIG 25. Also, a plurality of shapes, each of which is a shape of a half speech balloon, may be continued as reference layers 24.

Reference layer 24 may be formed into a shape of a half speech balloon when seen from the third surface side of probe 12 as illustrated in (B) in FIG 25. Reference layer 24 may have a protrusion at a part of the upper surface of the shape of the half speech balloon. Also, reference layer 24 may be formed into a quadrangular shape when seen from the third surface side of probe 12.

Ag/AgCl paste 51 will be described. Ag/AgCl paste 51 may have viscosity within a range of equal to or greater than 0.1 Pa·s and equal to or less than 300 Pa·s, for example. More preferably, Ag/AgCl paste 51 may have viscosity within a range of equal to or greater than 1 Pa·s and equal to or less than 100 Pa·s. Further preferably, Ag/AgCl paste 51 may have viscosity within a range of equal to or greater than 10 Pa·s and equal to or less than 50 Pa·s.

In order to cause Ag/AgCl paste 51 to have predetermined viscosity, an organic solvent such as thinner that contains, as a main component, toluene, xylene, ethyl acetate, butyl acetate, or acetone, for example.

An exposure size of reference layer 24 will be described. The height of the exposed surface of reference layer 24 may be within a range of equal to or greater than 1 µm and equal to or less than 200 µm, for example, in terms of curbing of decomposition of reference layer 24 over a long period of time. More preferably, the height of the exposed surface of reference layer 24 may be within a range of equal to or greater than 2 µm and equal to or less than 50 µm. Further preferably, the height of the exposed surface of reference layer 24 may be within a range of equal to or greater than 5 µm and equal to or less than 15 µm.

Also, the width of the exposed surface of reference layer 24 may be within a range of equal to or greater than 1 µm and equal to or less than 1,000 µm, for example. More preferably, the width of the exposed surface of reference layer 24 may be within a range of equal to or greater than 50 µm and equal to or less than 800 µm. Further preferably, the width of the exposed surface of reference layer 24 may be within a range of equal to or greater than 100 µm and equal to or less than 600 µm. Also, the exposure area of reference layer 24 may be within a range of equal to or greater than 0.5 µm² and equal to or less than 160,000 µm², for example. More preferably, the exposure are of reference layer 24 may be within a range of equal to or greater than 70 µm² and equal to or less than 32,000 µm². Further preferably, the exposure area of reference layer 24 may be within a range of equal to or greater than 390 µm² and equal to or less than 7,100 µm².

As described above, probe 12 of sensor 1 that is to be inserted into a living body to measure an analyte includes substrate 21, electrode 22 that is formed on substrate 21, and reference layer 24 that is formed on electrode 22. Reference layer 24 has the first surface covered with film 25 and has a third surface exposed.

In this manner, sensor 1 is adapted such that the first surface of reference layer 24 is covered with film 25, the third surface is exposed, the area in which reference layer 24 comes into contact with the analyte can be thus reduced, and decomposition of reference layer 24 (decomposition of Ag/AgCl) is curbed. Therefore, sensor 1 can measure the analyte with higher accuracy.

Also, reference layer 24 is applied to electrode 22 by the application technique and has a shape narrowed in the direction from the third surface toward the inside of reference layer 24. Therefore, reference layer 24 can be stably minutely formed in the first surface by the application technique.

Note that reference layer 24 can be further minutely formed if a sharp part of the Ag/AgCl paste with substantially a water drop shape or substantially a pointed arch shape, for example, applied to electrode 22 is disposed outside the product (outside probe 12) through a squeegee operation or the like.

Although reference layer 24 is assumed to be formed on electrode 22 by the application technique, reference layer 24 may be formed by the screen printing technique. Also, reference layer 24 is more preferably performed by the application technique out of the application technique and the screen printing technique. The reason is because the screen printing technique leads to a large loss of the material (Ag/AgCl), requires a measure against clogging of the screen printing plate due to the Ag/AgCl paste or the like, and requires complicated management for the manufacturing.

Although the embodiments have been described with reference to the accompanying drawings, the present disclosure is not limited to such examples. It is obvious for those skilled in the art that various modification examples or amendment examples can be achieved within the scope described in the claims. It should be understood that such modification examples and amendment examples also belong to the technical scope of the present disclosure.

The components in the embodiments may be arbitrarily combined without departing from the gist of the present disclosure.

For example, trimmed portions may be formed in probe 12 described in Embodiment 2 similarly to probe 12 described in Embodiment 1.

Also, probe 12 described in Embodiment 2 may include a protective film that covers reagent layer 23.

A part of film 25 of probe 12 described in Embodiment 2 may overlap reagent layer 23 at both ends of reagent layer 23 in the insertion direction as described in FIG 10. Also, film 25 on the side of the insertion direction may not be formed.

Also, a material similar to that of each component in probe 12 described in Embodiment 1 may be used as a material of each component in probe 12 described in Embodiment 2. The size of probe 12 described in Embodiment 2 may be similar to that of probe 12 described in Embodiment 1.

### Industrial Applicability

The present disclosure is suitable for use in a biosensor such as a CGM sensor, for example.

### Reference Signs List

1 Sensor
2 Living body
11 Main body
12 Probe
21 Substrate
22 Electrode
22a Working electrode
22b Reference electrode
22c Counter electrode
23 Reagent layer
24 Reference layer
25 Film
31 Upper surface
32 Back surface
33, 34 Side surface
35, 36 Trimmed portions
51 Ag/AgCl paste
61 Application apparatus
62 Nozzle

## Claims

1. A sensor (1) that measures an analyte, comprising:
a probe (12) that is to be inserted into a living body (2),
wherein the probe (12) includes
a substrate (21),
an electrode (22) that is formed on or above the substrate (21), and
a reference layer (24) that is formed on or above the electrode (22), and
the reference layer (24) has an upper surface covered with a film (25) and **characterized by** having only one side surface exposed.

2. The sensor (1) according to claim 1, wherein the reference layer (24) has a shape narrowed in a direction from the side surface toward inside of the reference layer (24) when seen from the upper surface.

3. The sensor (1) according to claim 1, wherein a shape of the reference layer (24) in a direction from the side surface toward inside of the reference layer (24) is an arc shape, an elliptical arc shape, or a triangular shape.

4. The sensor (1) according to claim 3, wherein the reference layer (24) has a plurality of the arc shapes, a plurality of the elliptical arc shapes, or a plurality of the triangular shapes.

5. The sensor (1) according to claim 1, wherein the side surface of the reference layer (24) is exposed to the analyte.

6. The sensor (1) according to claim 5, wherein the side surface of the reference layer (24) is covered with a protective film (25) through which the analyte is transmitted.

7. A method for manufacturing a sensor (1) that includes a probe (12) that is to be inserted into a living body (2) to measure an analyte, the method comprising:
manufacturing the probe (12) by
forming an electrode (22) on or above a sheet-shaped substrate (21);
forming a reference layer (24) on or above the electrode (22);
disposing a film (25) on or above the reference layer (24); and
cutting the sheet-shaped substrate (21) into a shape of the probe (12),
wherein the reference layer (24) is formed by applying a paste-form material ejected from a nozzle to the electrode (22),
a part of the reference layer (24) is cut when the sheet-shaped substrate (21) is cut, and
the reference layer (24) **characterized by** having only one side surface exposed.

## Patentansprüche

1. Sensor (1), der einen Analyten misst, mit:
einer Sonde (12), die in einen lebenden Körper (2) einzuführen ist,
wobei die Sonde (12) umfasst
ein Substrat (21),
eine Elektrode (22), die auf oder über dem Substrat (21) ausgebildet ist, und eine Referenzschicht (24), die auf oder über der Elektrode (22) ausgebildet ist, und
wobei die Referenzschicht (24) eine Oberseite aufweist, die mit einer Folie (25) bedeckt ist, **dadurch gekennzeichnet, dass** die Referenzschicht (24) nur eine freiliegende Seitenfläche aufweist.

2. Sensor (1) nach Anspruch 1, wobei die Referenzschicht (24) eine Form aufweist, die sich in einer Richtung von der Seitenfläche zum Inneren der Referenzschicht (24) hin von der Oberseite aus betrachtet verjüngt.

3. Sensor (1) nach Anspruch 1, wobei eine Form der Referenzschicht (24) in einer Richtung von der Seitenfläche zum Inneren der Referenzschicht (24) hin eine Bogenform, eine elliptische Bogenform oder eine dreieckige Form aufweist.

4. Sensor (1) nach Anspruch 3, wobei die Referenzschicht (24) mehrere der Bogenformen, mehrere der elliptischen Bogenformen oder mehrere der dreieckigen Formen aufweist.

5. Sensor (1) nach Anspruch 1, wobei die Seitenfläche der Referenzschicht (24) dem Analyten ausgesetzt ist.

6. Sensor (1) nach Anspruch 5, wobei die Seitenfläche der Referenzschicht (24) mit einer Schutzfolie (25) bedeckt ist, durch die der Analyt hindurchtritt.

7. Verfahren zur Herstellung eines Sensors (1), der eine Sonde (12) umfasst, die in einen lebenden Körper (2) einzuführen ist, um einen Analyten zu messen, wobei das Verfahren umfasst:
Herstellen der Sonde (12) durch
Bilden einer Elektrode (22) auf oder über einem blattförmigen Substrat (21);
Bilden einer Referenzschicht (24) auf oder über der Elektrode (22);
Anordnen einer Folie (25) auf oder über der Referenzschicht (24); und
Schneiden des blattförmigen Substrats (21) in eine Form der Sonde (12),
wobei die Referenzschicht (24) durch Aufbringen eines Pasten-förmiges Materials, das aus einer Düse ausgestoßen wird, auf die Elektrode (22) gebildet wird, und
wobei ein Teil der Referenzschicht (24) geschnitten wird, wenn das blattförmige Substrat (21) geschnitten wird,
**dadurch gekennzeichnet, dass** die Referenzschicht (24) nur eine freiliegende Seitenfläche aufweist.

## Revendications

1. Capteur (1) qui mesure un analyte, comprenant :
une sonde (12) qui est destinée à être insérée dans un corps vivant (2),
dans lequel ladite sonde (12) comprend
un substrat (21),
une électrode (22) qui est formée sur ou au-dessus du substrat (21), et
une couche de référence (24) qui est formée sur ou au-dessus de l'électrode (22), et
dans lequel ladite couche de référence (24) présente une surface supérieure qui est recouverte d'un film (25), **caractérisé par le fait que** la couche de référence (24) présente seulement une surface latérale exposée.

2. Capteur (1) selon la revendication 1, dans lequel la couche de référence (24) présente une forme qui, vue depuis la surface supérieure, se rétrécit dans une direction de la surface latérale vers l'intérieur de la couche de référence (24).

3. Capteur (1) selon la revendication 1, dans lequel une forme de la couche de référence (24), dans une direction de la surface latérale vers l'intérieur de la couche de référence (24), présente une forme d'arc, une forme d'arc elliptique ou une forme triangulaire.

4. Capteur (1) selon la revendication 3, dans lequel la couche de référence (24) présente une pluralité des formes d'arc, une pluralité des formes d'arc elliptique ou une pluralité des formes triangulaires.

5. Capteur (1) selon la revendication 1, dans lequel la surface latérale de la couche de référence (24) est exposée à l'analyte.

6. Capteur (1) selon la revendication 5, dans lequel la surface latérale de la couche de référence (24) est recouverte d'un film protecteur (25) à travers lequel l'analyte est transmis.

7. Procédé de fabrication d'un capteur (1) qui comprend une sonde (12) laquelle est destinée à être insérée dans un corps vivant (2) pour mesurer un analyte, le procédé comprenant :
fabriquer la sonde (12) en :
formant une électrode (22) sur ou au-dessus d'un substrat en forme de feuille (21) ;
formant une couche de référence (24) sur ou au-dessus de l'électrode (22) ;
déposant un film (25) sur ou au-dessus de la couche de référence (24) ; et
découpant le substrat en forme de feuille (21) selon une forme de la sonde (12),
dans lequel la couche de référence (24) est formée par application d'un matériau sous forme de pâte éjecté d'une buse, sur l'électrode (22), et
dans lequel une partie de la couche de référence (24) est découpée lors de la découpe du substrat en forme de feuille (21), et
**caractérisé par le fait que** la couche de référence (24) présente seulement une surface latérale exposée.
